# EUROPEAN PATENT APPLICATION

(11) **EP 2 220 994 A1**
(43) Date of publication of application: **25.08.2010**
(21) Application number: 08858638.3
(22) Date of filing: 26.11.2008
(51) Int. Cl.: A61B 5/00, A61B 5/145, H05K 5/02

(54) **MEDICAL DEVICE**

(30) Priority: 10.12.2007 JP 2007318318
(71) Applicant: ARKRAY, Inc., Minami-ku Kyoto-shi Kyoto 601-8045 (JP)
(72) Inventor: NISHIMURA, Hideki, Kyoto-shi Kyoto 601-8045 (JP); KUBO, Masayuki, Kyoto-shi Kyoto 601-8045 (JP); OHAMA, Shinji, Toon-shi Ehime 791-0395 (JP)
(74) Representative: Mathys & Squire LLP
(86) International application number: PCT/JP2008/003473
(87) International publication number: WO 2009/075065

(57) **Abstract**

In a medical device (1) that measures a condition of a living body, a recess (8) is formed on the exterior (surfaces (6) and (7)) of its casing (2). An anti-slip member (3) is attached to the inside of the recess (8). Moreover, another recess (9) is provided inside the recess (8). The recess (9) is formed such that an end (10) of the recess (9) protrudes from the anti-slip member (3) when the anti-slip member (3) is attached to the inside of the recess (8). In the case where a display screen (4) that displays a measurement result is provided on the casing (2), it is preferable that the surfaces (6) and (7) are adjacent to a surface (5) on which the display screen (4) is provided.

## Description

### Technical Field

The present invention relates to medical devices, and particularly to a portable medical device.

### Background Art

Recently, the size reduction of medical devices has advanced, and the size of glucose meters, sphygmomanometers, pulse rate meters and the like, for example, has been reduced to such a degree that they can be portable. Such medical devices are often carried around by patients, medical personnel and the like, and their frequency of use is high. In particular, glucose meters are essential medical devices for diabetic patients, and the frequency of carrying and the frequency of use are both very high.

As a precision device, such portable medical devices are vulnerable to impacts and caution should be exercised in handling such devices not to drop. Consequently, protective covers have been conventionally proposed to protect medical devices from damages when the devices are dropped (see for example, Patent document 1).

Moreover, a protective cover disclosed in Patent document 1 prevents dropping itself by having an anti-slip member on a side surface. Specifically, the anti-slip member is a member that is provided with recesses and projections on the surface and is formed of a rubber or a resin. Moreover, the anti-slip member may be formed integrally with the protective cover.
Patent document 1: Japanese Patent Application Publication No. 2004-313269

### Disclosure of Invention

### Problem to be Solved by Invention

Incidentally, the anti-slip member deteriorates or abrades away when used for a long time, and the anti-slipping effect is decreased. Thus the anti-slip member needs to be changed regularly.

However, according to the protective cover disclosed in the above Patent document 1, the anti-slip member is embedded in the side surface of the protective cover, and changing the anti-slip member requires enormous efforts. Furthermore, when the anti-slip member is attached with an adhesive, adhesion may increase over time, which makes changing even harder. Moreover, the anti-slip member may be formed integrally with the protective cover. In such a case, the protective cover needs to be changed with the anti-slip member, resulting in increase in an economic burden on users.

Moreover, when the protective cover is attached to a medical device, the size of the medical device is increased by the size of the protective cover, which prevents the progress of size reduction of medical devices. Thus it is required to provide a medical device with the anti-slipping effect without attaching a protective cover.

In contrast, simply attaching the anti-slip member to a casing of the medical device seems to allow one to change the anti-slip member easily, without a need for a protective cover. In such a case, however, since the edge of the anti-slip member easily comes into contact with an external object, the anti-slip member is easily detached, and thus a user needs to attach again frequently.

It is an object of the present invention to provide a medical device that can solve the above-described problem, and can facilitate change of the anti-slip member by a user, while preventing an anti-slip member attached to a casing from easily getting detached.

### Means for Solving Problem

In order to achieve the above-described object, a medical device according to the present invention is a medical device that measures a condition of a living body, including a casing having a first recess in the exterior and an anti-slip member, wherein the anti-slip member is attached to an inside of the first recess, the first recess includes a second recess inside, and the second recess is formed such that an end thereof protrudes from the anti-slip member attached to the inside of the recess.

With a medical device according to the present invention, since the anti-slip member is attached to the inside of the first recess provided for the casing, the edge of the anti-slip member does not easily come into contact with an external object. Accordingly, durability of the anti-slip member is significantly enhanced unlike in the case where the anti-slip member is simply attached to the surface of the casing, and the occurrence of detachment unintended by the user is prevented. Moreover, with a medical device according to the present invention, since the anti-slip member is easily detached by inserting a stick-like member or the like to the second recess, a user can easily change the anti-slip member. Furthermore, as a result, decrease in the anti-slipping effect is prevented.

In a preferred embodiment of the above-described medical device of the present invention, the casing is provided with a display screen that displays a measurement result, and the first recess is provided in a surface adjacent to a surface where the display screen is provided. With the above-described embodiment, since the anti-slip member is disposed at a position with which the hand of the user is likely to come into contact, the possibility of accidental dropping by the user will be further suppressed particularly when the medical device is portable.

### Effects of the Invention

As stated above, with a medical device according to the present invention, an anti-slip member attached to the casing can be prevented from easily getting detached (occurrence of detachment unintended by the user is prevented). In contrast, when the user intends to detach the anti-slip member, the anti-slip member can be easily detached, and thus change of the anti-slip member by the user is facilitated.

### Brief Description of Drawings

[FIG. 1] FIG. 1 is a perspective view illustrating the configuration of a medical device according to an embodiment of the present invention, showing a state in which an anti-slip member is not attached to a casing of the medical device.
[FIG. 2] FIG. 2 is a perspective view illustrating the configuration of the medical device according to the embodiment of the present invention, showing a state in which an anti-slip member is attached to the casing of the medical device.
[FIG. 3] FIG. 3 is a cross-sectional view taken along the cutting-plane line A-A' shown in FIG. 2.
[FIG. 4] FIG. 4 is a side view illustrating an enlarged side surface of the medical device shown in FIG. 2.
[FIG. 5] FIG. 5 is a perspective view illustrating an operation for detaching the anti-slip member.

### Description of Reference Numerals

- 1: medical device
- 2: casing
- 3: anti-slip member
- 4: display screen
- 5: principal surface of casing
- 6, 7: surfaces (side surfaces) adjacent to principal surface
- 8: recess
- 9: recess (for detachment)
- 10: end of recess for detachment
- 11: adhesive material layer
- 12: stick-like member

### Best Mode for Carrying Out the Invention

### (Embodiment)

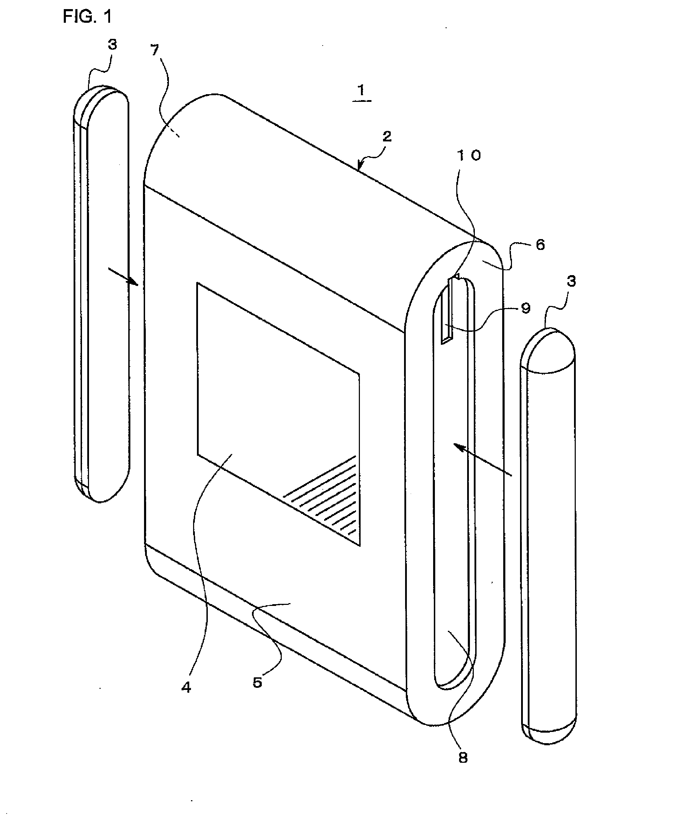

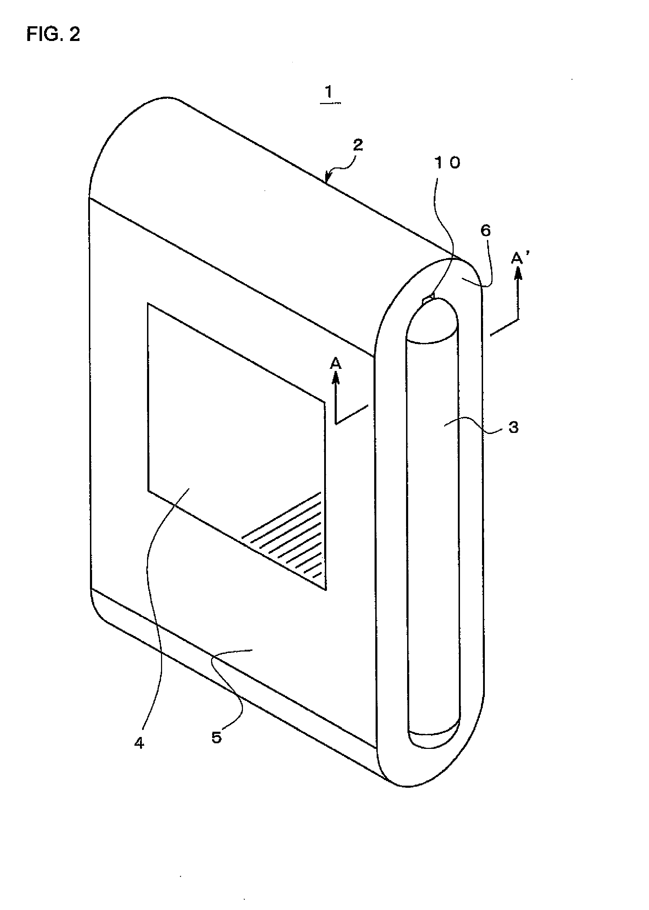

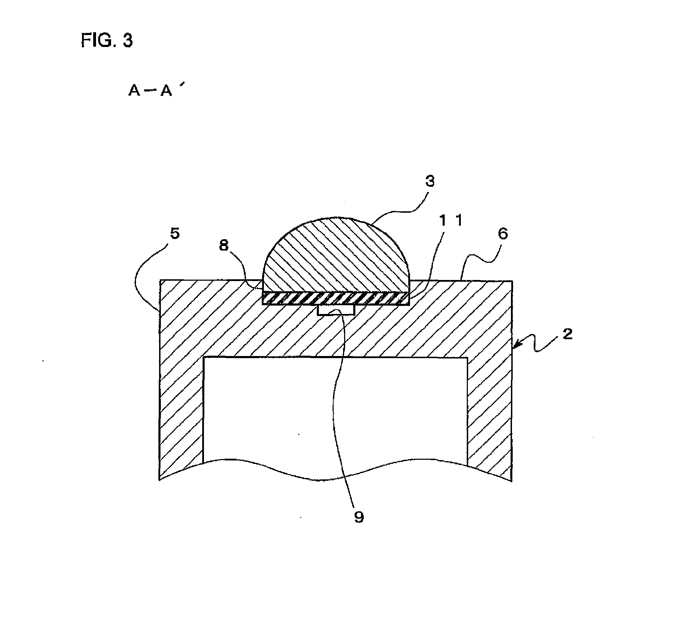

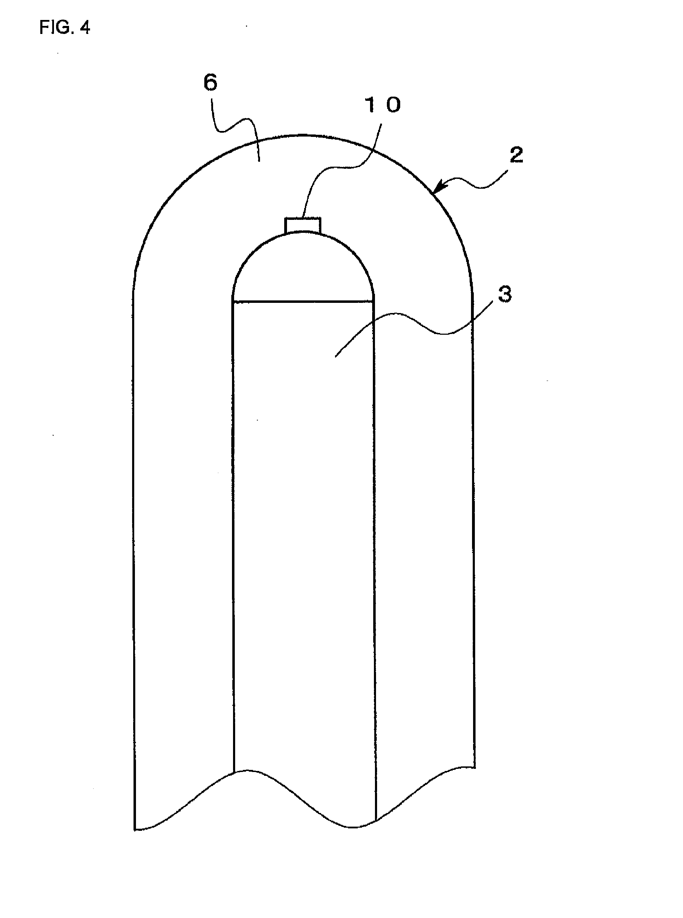

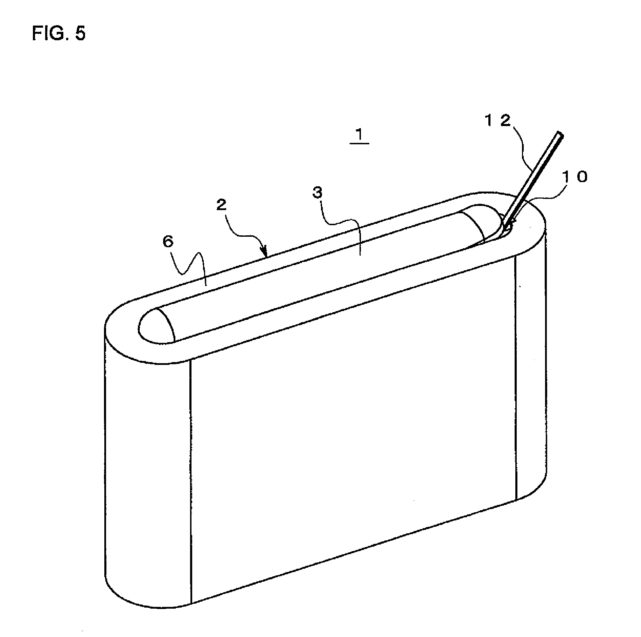

A medical device according to an embodiment of the present invention is described referring to FIGS. 1 to 5. FIGS. 1 and 2 are perspective views illustrating the configuration of a medical device according to an embodiment of the present invention. FIG. 1 shows a state in which an anti-slip member is not attached to a casing of the medical device, and FIG. 2 shows a state in which an anti-slip member is attached to the casing of the medical device. FIG. 3 is a cross-sectional view taken along the cutting-plane line A-A' shown in FIG. 2. FIG. 4 is a side view illustrating an enlarged side surface of the medical device shown in FIG. 2. FIG. 5 is a perspective view illustrating an operation for detaching the anti-slip member.

As shown in FIGS. 1 and 2, a medical device 1 according to the present embodiment is a medical device that measures a condition of a living body, including, for example, a glucose meter, a sphygmomanometer, a lactate meter, a ketone body measuring device, a thermometer, a urine analyzer, and a lipid measuring device. Moreover, the medical device 1 is configured in a hand-held size, and is assumed to be carried by a user such as a patient.

The medical device 1 includes a casing 2. As shown in FIG. 1, the casing 2 is provided with a recess 8 on its exterior. In the present embodiment, a display screen 4 that displays measurement results, including, for example, a blood glucose level and a blood pressure value is provided on one principal surface 5 of the exterior of the casing 2. Then, a recess 8 is formed in each of the surfaces (side surfaces) 6 and 7 that are adjacent to the principal surface 5. Moreover, as shown in FIGS. 2 and 3, an anti-slip member 3 is attached to the inside of the recesses 8. It should be noted that the illustration of the recess formed in the external surface 7 has been omitted in FIGS. 1 and 2.

Since the anti-slip member 3 is attached to the casing 2 of the medical device 1 as described above, the occurrence of a situation where the user drops the device by accident is suppressed. Moreover, in the present embodiment, the anti-slip member 3 is provided on the side surfaces, i.e., the surfaces 6 and 7, with which the hand of the user can most easily come into contact when the user operates the medical device 1, which enhances the anti-slipping effect.

Furthermore, since the anti-slip member 3 is attached to the inside of the recess 8, the anti-slip member 3 is detached less easily than the case where the anti-slip member 3 is simply attached to the exterior of the casing 2. That is, since the edge of the anti-slip member 3 is not likely to come into contact with an external object, detachment of the anti-slip member 3 unintended by the user is prevented.

Moreover, in the present embodiment, the anti-slip member 3 is formed of a resin material or a rubber material. The resin material and the rubber material may be any material that has a high coefficient of friction on the surface. Furthermore, the anti-slip member 3 may be formed of a member whose coefficient of friction has been increased by forming recesses and projections on the surface, and a material other than the resin material or the rubber material may be used in such a case.

Specific examples of the resin material for forming the anti-slip member 3 include resin materials such as acrylate resins, urethane resins, polyethylene resins, polypropylene resins, ABS resins, polyvinyl chloride resins, AS resins, epoxy resins, and UV curable resins. Moreover, examples of the rubber material for forming the anti-slip member 3 include rubber materials such as silicone rubbers, butadiene rubbers, isoprene rubbers, chloroprene rubbers, butyl-rubbers, fluorocarbon rubbers, and styrene-butadiene rubbers.

Furthermore, as shown in FIG. 3, the anti-slip member 3 is attached to the casing 2 with an adhesive material layer 11 according to the present embodiment. There is no particular limitation with respect to the formation material, the thickness and the like of the adhesive material layer 11. As the formation material of the adhesive material layer 11, it is possible to use a material appropriately selected from the existing materials according to the formation material of the casing. The thickness of the adhesive material layer 11 may be appropriately selected such that the desired adhesion can be achieved. It should be noted that the adhesive material layer 11 can be formed by applying an adhesive material beforehand. A double-stick tape may be used as a substitute for the adhesive material layer 11.

Moreover, as shown in FIGS. 1, 3 and 4, according to the present embodiment, another recess 9 is provided inside the recess 8 in the casing 2 of the medical device 1. The recess 9 is formed such that an end 10 of the recess 9 protrudes from the anti-slip member 3 when the anti-slip member 3 is attached to the inside of the recess 8 (see FIG. 4). It should be noted that one end (upper end in the figure) of the ends of the recess 8 agrees with the end 10 of the recess 9, and also protrudes from the anti-slip member 3.

Consequently, as shown in FIG. 5, according to the present embodiment, the anti-slip member 3 can be easily detached by inserting, for example, a stick-like member 12 to the recess 9. Thus a user can easily change the anti-slip member 3. Moreover, since change of the anti-slip member 3 is facilitated, decrease in the anti-slipping effect is prevented. It should be noted that the size of the area of the recess 9 that protrudes from the anti-slip member is not limited but may be such an extent that the user's fingertip can inserted.

### Industrial Applicability

As described above, the present invention is effective for improvement in handling a medical device, in particular, a small portable medical device. The medical device according to the present invention has industrial applicability.

## Claims

1. A medical device that measures a condition of a living body, comprising:
a casing having a first recess in the exterior; and
an anti-slip member, wherein
the anti-slip member is attached to an inside of the first recess,
the first recess includes a second recess inside, and
the second recess is formed such that an end thereof protrudes from the anti-slip member attached to the inside of the recess.

2. The medical device according to claim 1, wherein
the casing is provided with a display screen that displays a measurement result, and the first recess is provided in a surface adjacent to a surface where the display screen is provided.
